Europäisches Patentamt

(19) European Patent Office

Office européen des brevets

(11) Publication number : **0 509 834 A2**

# EUROPEAN PATENT APPLICATION

(21) Application number : **92303484.7**

(22) Date of filing : **16.04.92**

(51) Int. Cl.⁵ : **C12N 1/20**, C12P 17/18,
// (C12N1/10, C12R1:38)

(30) Priority : **17.04.91 JP 112471/91**

(43) Date of publication of application :
**21.10.92 Bulletin 92/43**

(84) Designated Contracting States :
**CH DE FR GB IT LI**

(71) Applicant : **AMANO PHARMACEUTICAL CO.,
LTD.**
**2/7, Nishiki 1-chome, Naka-ku**
**Nagoya-shi Aichi-ken (JP)**

(72) Inventor : **Koide, Yoshinao, c/o Amano
Pharm.Co.,Ltd.**
**51, Nishi Shiroyashiki, Ohaza Kunotsubo
Nishiharumachi, Nishi Kasugai-gun, Aichi (JP)**
Inventor : **Nakane, Seiji, c/o Amano
Pharm.Co.,Ltd.**
**51, Nishi Shiroyashiki, Ohaza Kunotsubo
Nishiharumachi, Nishi Kasugai-gun, Aichi (JP)**
Inventor : **Suzuoki, Michihiro, c/o Amano
Pharm.Co.,Ltd.**
**51, Nishi Shiroyashiki, Ohaza Kunotsubo
Nishiharumachi, Nishi Kasugai-gun, Aichi (JP)**

(74) Representative : **West, Alan Harry et al
R.G.C. Jenkins & Co. 26 Caxton Street
London SW1H 0RJ (GB)**

(54) **Novel bacterial strain and method for producing theobromine using the same.**

(57)   The present invention provides a method to efficiently produce theobromine from caffeine using a bacterial mutant constitutively metabolizing caffeine and lacking the ability to demethylate theobromine into 7-methylxanthine. The double mutant strain is derived from a strain of the genus Pseudomonas which can convert caffeine to theobromine constitutively, and accumulate theobromine in culture broth without the nickel ion.

EP 0 509 834 A2

## BACKGROUND OF THE INVENTION

### Field of the Invention

The present invention relates to a method for producing theobromine. More specifically, the present invention relates to a method for producing theobromine in large scale, comprising modifying a microorganism assimilating caffeine, and thereby efficiently converting caffeine to theobromine.

Theobromine (3,7-dimethylxanthine) is a demethylated compound of caffeine (1,3,7-trimethylxanthine), and is useful as a diuretic and an intermediate of pharmaceutical agents.

### Related Background Art

Conventionally theobromine has been produced by extraction from cacao beans or by synthesis from 3-methyl urea. Microorganisms such as Bacillus coagulans, Stemphylium sp., Aspergillus niger, species of the genus Pseudomonas and the like, are known to metabolize and decompose caffeine [Experientia, 27, 481(1971); Nippon Nogeikagaku Kaishi, 45, 378(1971); J. Bacteriol., 123, 1088(1975); Hoope-Seyler's Z. Physiol. Chem., 358, 807(1977); Hoope-Seyler's Z. Physiol. Chem., 361, 1763(1980)].

As a practical method to convert caffeine to theobromine using microorganisms, there is a method to use a microorganism of the genus Pseudomonas isolated from soil, in the presence of nickel ion (Japanese Laid-open Patent Application No. 59-210896).

There is also an attempt to convert caffeine into theobromine using a mutant of a microorganism which can assimilate caffeine [Appl. Microbiol. Biotechnol., 25, 334(1987)].

However, the prior art (Japanese Laid-open Patent Application No. 59-210896) has many problems from the respect of industrial-scale production. For example, the conversion efficiency of caffeine into theobromine is insufficient so that a considerable amount of caffeine is left unreacted and the yield of theobromine is about 15 mg/ml, and nickel ion added for the improvement of the conversion efficiency should be recovered from the waste after the production of theobromine.

The conversion method of caffeine into theobromine using a mutant bacterium is far from the industrial production of theobromine, because the yield of theobromine is as low as 0.2 mg/ml along with the co-production of heteroxanthine.

The present inventors have carried out intensive investigations under such circumstances. Consequently, it is inferred that the effect of the nickel ion in the prior art (Japanese Laid-open Patent Application No. 59-210896) is the inhibition of the conversion of theobromine to 7-methylxanthine, resulting in the remarkable accumulation of theobromine.

Then, investigation has been carried out over the metabolic pathway of caffeine in a microorganism of the genus Pseudomonas, newly isolated from natural source and having the potency to assimilate caffeine. It is deduced that caffeine is sequentially demethylated and converted into theobromine, 7-methylxanthine, then to xanthine. Furthermore, when 7-methylxanthine is added to a nutrient culture medium containing caffeine, caffeine is metabollized much rapidly. That means the caffeine metabolic pathway of this strain is induced by 7-methylxanthine.

Based on the findings described above, a microorganism of genus Pseudomonas, isolated from natural source and having the potency of assimilating caffeine, was mutagenized to obtain a mutant strain capable of metabolizing caffeine constitutively. Then, the further mutagenization brought about a double mutated strain which could not demethylate theobromine into 7-methylxanthine. By using the double mutated strain, caffeine can be converted efficiently into theobromine for accumulation of the latter without the presence of nickel ions.

Using this strain, furthermore, caffeine or a medium component containing caffeine is continuously added to compensate the decrease of caffeine, whereby the conversion of caffeine into theobromine can be facilitated continuously in a steady state. Consequently, a remarkable amount of theobromine can be produced and accumulated during the culture.

## SUMMARY OF THE INVENTION

The present invention relates to a mutant bacterial strain derived by inducing mutations in a microorganism of genus Pseudomonas having the potency to convert caffeine into theobromine, the mutant strain is capable of constitutively converting caffeine into theobromine and genetically lacks the system for the demethylation of theobromine. The present invention also relates to a method for converting caffeine into theobromine comprising continuous addition of caffeine into the culture and recovering the accumulated theobromine in a large quantity, using the above microorganism.

## DETAILED DESCRIPTION OF THE PREFERRED EMBODIMENTS

The parent strain to be used in the present invention includes a wide variety of microorganisms having the caffeine resistance and the potency to convert caffeine into theobromine. More preferably, such microorganisms are of the genus Pseudomonoas, and the mutant strains of these microorganisms can be used in the present invention.

Specifically the mutant strains are derived from the parent strain Pseudomonas putida IF-3 isolated from soil.

The induction of mutation into microorganisms can be carried out by known methods. There may be employed, for example, the chemical treatment using N-methyl-N′-nitro-N-nitrosoguanidine (hereinafter referred as "NTG") as a mutagenizing agent.

As such mutation inducing agents, there may be used 2-aminopurine, 5-bromouracil, ethyl methanesulfonate, dimethyl sulfate, acriflavine, acridine orange, hydrazine, 4-nitroquinoline-N-oxide, manganese chloride, etc. Furthermore, physical means using ultra-violet irradiation, radioactive irradiation of X ray, γ ray, etc. and the like may be also employed.

For the isolation of the mutant strains, known procedures may be applied. There may be applied direct methods such as culturing mutagenized microorganisms and examining each colony for the presence of mutation, or the replica method as the modification of the above method, condensation method using antibiotics such as penicillin, the suicide method using specific substrates, and methods in an appropriate combination thereof.

According to the present invention, experimental procedures are as follows; about 100 caffeine-resistant bacteria were separated from soil and were cultured in an agar medium containing 1.0 % Bacto-pepton, 0.5 % Bacto-yeast extract, 0.5 % sodium chloride, and 2.0 % caffeine. The strains were inoculated into an agar medium, pH 7.0 containing 0.3 % caffeine, 0.3 % ammonium sulfate, 0.5 % dipotassium phosphate, 0.1 % sodium chloride, and 0.2 % magnesium sulfate, and then cultured at 30°C for three days to select strains showing excellent growth.

The bacteriological properties of the selected strain are as follows;

a. Morphological properties

1. Gram staining; negative
2 form and size of cell; rod of about 0.8 x 2 to 3 μm
3. presence of motility; positive
4. flagellum; > 1
5. formation of spore; negative

b. Physiological properties

1. O-F test (Hugh-Leifson method); aerobic acid-production
2. hemolysis; positive
3. oxygen requirement; aerobic
4. acid production from sugars;

| D-glucose | + |
|-----------|---|
| D-xylose | + |
| mannitol | - |
| lactose | - |
| sucrose | - |
| D-maltose | - |
| D-fructose | - |
| L-arabinose | + |
| raffinose | - |
| inulin | - |
| salicine | - |
| Sepharose | - |
| D-sorbitol | - |
| D-galactose | + |
| glycerol | - |

5. catalase test; positive

6. oxidase test; positive

7. growth on McConkey's medium; positive

8. growth on SS agar medium; positive

9. hydrolysis of DBH; negative

10. accumulation of PHB; negative

11. utilization of citric acid; positive

12. hydrolysis of urea; negative

13. reduction of nitrates; positive

14. denitrification of nitrates; negative

15. heat resistance (at 60°C for 30 minutes); not resistant

16. growth temperature;

> growth at 5°C
>
> growth at 25°C
>
> growth at 37°C
>
> no growth at 42°C

17. hydrolysis of gelatin; negative

18. litmus milk reaction; positive

19. hydrolysis of starch; negative

20. hydrolysis of casein; negative

21. lectinase reaction; negative

22. decarboxylation of lysine; negative

23. arginine dihydrolase; positive

24. decarboxylation of ornithine; negative

25. hydrolysis of esculin; negative

26. NaCl resistance;

> resistant to 0 % NaCl
>
> resistant to 4 % NaCl
>
> not resistant to 6 % NaCl
>
> not resistant to 7 % NaCl

27. acylamidase; negative

28. oxidation of gluconic acid; negative

29. presence of DNAase; negative

30. pigment production;

> in King A medium; negative
>
> in King B medium; positive

31. hydrolysis of TW-80; negative

32. growth in NAC medium; positive

33. hydrolysis of agar; negative

34. levan production from sucrose; negative

35. deamination of phenylalanine; negative

36. growth in methylene-blue milk;

> decrease in methylene blue; positive
>
> coagulation; negative
>
> peptonization; negative

37. acid from TSI; negative/negative

38. production of hydrogen sulfide in TSI medium; negative

39. hydrolysis of sodium hippurate; positive

40. MR test; negative

41. VP test; negative

The bacteriological properties described above were examined on the basis of Manual of the Identification of Medical Bacteria (MIMB), Identification of Microorganisms (Society of Hygienic Technology, Japan), and Classification and Identification of Microorganisms (volume II) (Academic Society Press Center, Japan).

Based on the various bacteriological properties described above, investigation was carried out according to the description of Guide to Presumptive Identification and Bergey's Manual of Systematic Bacteriology. The present bacterial strain was identified as one species of Pseudomonas putida and named as Pseudomonas putida IF-3, from the results of the investigation over the number of flagellum, growth at 42°C, hydrolysis of gelatin and TW-80, acid production from trehalose and mannitol.

The present strain was deposited as FERM BP-3824 in the Patent Microorganism Depository, Fermentation Research Institute, Agency of Industrial Science and Technology at 1-3, Higashi 1-chome, Tsukuba-shi, Ibaragi-ken, 305, Japan.

The strain Pseudomonas putida F-3 is resistant to a high concentration of caffeine and can metabolize caffeine. The strain can grow, using theobromine or 7-methylxanthine as a sole carbon source, but cannot grow on 1-methylxanthine, 3-methylxanthine, or theophylline as a sole carbon source. This finding indicates that the caffeine metabolism of the present strain is via the sequential pathway from caffeine to theobromine, 7-methylxanthine and then to xanthine.

When the mutant strain incapable of demethylating theobromine into 7-methylxanthine, derived from strain IF-3, is cultured in a medium containing caffeine, no conversion of caffeine is observed, but when 7-methylxanthine is added to the same medium, the conversion of caffeine into theobromine occurs so that the accumulation of theobromine is observed. This indicates that caffeine demethylase of the strain IF-3 is an enzyme inducible by 7-methylxanthine.

Based on the findings described insofar, the present inventors have subjected the parent strain Pseudomonas putida IF-3 to mutagenization treatment and isolated a mutant bacterial strain Pseudomonas putida IF-3-9C-21 (referred as "9C-21" hereinafter) which constitutively converts caffeine into theobromine without converting theobromine into 7-methylxanthine.

The present strain was deposited as FERM BP-3825 in the Patent Microorganism Depository, Fermentation Research Institute, Agency of Industrial Science and Technology at 1-3, Higashi 1-chome, Tsukuba-shi, Ibaragi-ken, 305, Japan.

When the 9C-21 strain was cultured in a medium, pH 6.7 consisting 0.9 % caffeine, 1.0 % Bactotrypton and 0.5 % Bacto-extract at 30°C overnight, among the xanthine derivertives only theobromine was produced in the culture broth at an amount of 7.5 mg/ml.

As above, caffeine could be converted into theobromine at high efficiency. Further investigation was carried out to increase the accumulated amount of theobromine. As a result, a greater amount of theobromine could be accumulated, by sequentially adding caffeine in a culture broth after the cell growth, thereby effecting the continuous conversion of caffeine into theobromine.

As the nutrient medium to culture the mutant strain of the present invention, there may be used either of natural medium or synthetic medium, containing routine components such as carbon sources in addition to caffeine, nitrogen sources and inorganic salts, and if the mutant strain requires, other nutrients, auxiliary components (pH regulator, emulsifier, antifoaming agent, etc.) and the like can be added. The other carbon sources may be for example, sugars such as glucose, xylose, arabinose, etc., sugar alcohols such as glycerol, sorbitol, etc., organic acids such as citric acid, fumaric acids, etc. The nitrogen sources, there may include inorganic nitrogen sources such as ammonium salt, nitrate, etc.; and organic nitrogen sources such as meat extract, yeast extract, trypton, caltivator, etc. As inorganic salts, thereby may be used potassium phosphate, potassium chloride, magnesium sulfate, sodium chloride, sodium phosphate, ferrous sulfate, etc.

The concentration of caffeine is 0.1 to 2.5 %, and preferably 0.5 to 1.0 %. The culture is carried out at a temperature of 10 to 40°C and pH of about 4.5 to 9.0; preferably at a temperature of 20 to 30°C and pH of 6.5 to 7.5. The culture is preferably continued for 5 to 20 hours. By continuously feeding the medium containing a carbon source(s) such as glucose, xylose, etc. and nitrogen source(s) such as meat extract, yeast extract, sodium glutamate, etc., and by simultaneously adding caffeine so that the caffeine concentration in the medium might be 0.1 mg/ml to 30 mg/ml, preferably 5 mg/ml to 20 mg/ml, the conversion reaction of caffeine to theobromine continueously occurs. The caffeine may be added as a solid or an aqueous solution.

In accordance with the present invention, there may be used any of the culture itself obtained by the culture method described above, the bacteria cells, the culture filtrate, the disrupted cells, the lyophilized cells, the cell extract with solvents such as ethanol, toluene, ether, etc., and the immobilized cells. By making the culture product and the like and caffeine come in contact in an aqueous medium the caffeine is converted into theobromine. Caffeine may be added as a solid, or an aqueous solution. Caffeine may be present in a suspension state exceeding 21.7 mg/ml, solubility thereof at room temperature. The contact of the culture product etc. with caffeine may be effected by the batch method, or by the continuous method using a column.

Under the conditions described above, such product(s) and caffeine are aerated for 50 to 80 hours for agitation to continue the conversion reaction under aerobic conditions. The produced theobromine is deposited as crystalline in the medium. The identification and qualification of theobromine thus produced, was carried out by thin-layer chromatography, liquid chromatography and the like.

The present invention will now be explained in experiments and examples hereinbelow, but the present invention is not limited to them.

EP 0 509 834 A2

## EXPERIMENT

Effect of nickel ion on <u>Pseudomonas sp</u>. 188-1 (FERM P-7073) described in Japanese Laid-open Patent Application No. 59-210896.

The bacterial strain described above was cultured at 28°C in a medium containing 0.35 % caffeine, 0.1 % ammonium sulfate, 0.5 % potassium phosphate, 0.01 % magnesium sulfate·7H$_2$O, and 0.3 % yeast extract. When the caffeine content in the medium reached zero, caffeine was added with or without the nickel ion as shown in Table 1 and the amount of the remaining caffeine and the accumulated amount of theobromine were measured subsequently. For 45 ml of the culture medium caffeine and nickel ion were added at levels of 1.7 g and 1 mM, respectively. Other nutrient sources, 0.5 g of sodium glutamate and 5 ml of phosphate buffer (pH 7.0), were also added and the reaction was carried out under agitation at 25°C for 40 hours.

As in shown in Table 1, the amount of theobromine accumulated is greater when the nickel ion was added.

However, the amount of the remaining caffeine was smaller when the nickel ion was not added. Therefore, it is suggested that theobromine is accumulated as a consequence of the inhibition of the demethylation of theobromine by the nickel ion.

## EXAMPLE 1

The bacterial strain <u>Pseudomonas putida</u> IF-3 (FERM P-12172) was cultured in TY medium containing 1.0 % Bacto-trypton and 0.5 % Bacto-yeast extract (Difco) to the logarithmic growth phase, and collected by centrifugation and washed. Subsequently, the cells were suspended in 5 ml of 50 mM Tris-maleate buffer (pH 6.0).

NTG was added to the cell suspension to 100 µg/ml, which was incubated at 30°C for 30 minutes. After the NTG treatment, cells were washed twice with a 0.9 % sodium chloride solution. The cells were suspended in TY medium and cultured with agitation at 30°C overnight. The cells were collected by centrifugation and washed twice with 0.9 % sodium chloride, followed by culturing in 5 ml of caffeine minimum medium (pH 7.0, 0.3 % ammonium sulfate, 0.5 % dipotassium phosphate, 0.1 % sodium chloride, 0.2 % magnesium sulfate·7 H$_2$O, and 0.1 % caffeine) at 30°C for six hours. Then, the resulting bacterial culture was diluted and plated on the caffeine minimum medium, pH 7.0 containing 0.3% ammonium sulfate, 0.5 % dipotassium phosphate, 0.1 % sodium chloride, 0.2 % magnesium sulfate·7 H$_2$O, 0.1 % caffeine, and 1.5 % agar. After the culture at 30°C for two days, colonies were screened for excellent growth.

Each mutant strain obtained was inoculated into LKC medium, pH 7.0 containing 1.0 % Bacto-trypton, 0.5 % Bacto-yeast, 0.5 % sodium chloride, 0.5 % dipotassium phosphate, and 0.1 % caffeine, for culture at 30°C for eight hours. Subsequently, the caffeine concentration in the medium was measured. A bacterial strain which showed a higher caffeine decrease compared with the parent strain was isolated as the constitutive mutant strain in caffeine metabolism, and named strain 9C.

Furthermore, strain 9C was treated with NTG in the same manner as described above. The bacterial cells were washed and cultured under agitation at 30°C overnight in a theobromine minimum medium containing 0.3 % theobromine and the same inorganic salts as in the caffeine minimum medium and carbenicillin 300 µg/ml. The cells were washed twice in 0.9 % NaCl, and diluted and plated on LT agar medium, pH 6.8 containing 1.0 % Bacto-trypton, 0.5 % Bacto-yeast extract, 0.3 % theobromine, and 1.5 % agar, and incubated at 30°C for two days to form colonies. Twenty five colonies, which did not decompose theobromine around the colonies were obtained.

The assimilation properties for caffeine, theobromine, and 7-methylxanthine of the isolated strains were examined, and subsequently strain 9C-21 which assimilated only 7-methylxanthine was isolated. The strain 9C-21 is a double mutant which constitutively metabolizes caffeine but does not convert theobromine into 7-methylxanthine.

## EXAMPLE 2

Strain 9C-21 (FERM P-12173) was inoculated in a 500-ml Sakaguchi flask containing 75 ml of the medium (pH 7.0, 1.0 % caffeine, 1.0 % Bacto-trypton, 0.5 % Bacto-yeast extract, 0.5 % sodium chloride, 0.1 % glucose, 0.3 % dipotassium phosphate) and incubated at 30°C overnight. One percent of the resulting culture broth was inoculated in 3 liter of the above medium in a 5-liter mini-jar. The culture condition was as follows;
culture temperature; 30°C
agitating aeration; 1 vvm, 600 rpm
culture period; nine hours
reaction time; 39 hours
agitation paddle; flat-type paddles with six disk

6

turbines, arranged in two steps
feeding medium; 25 % glucose, and 2.5 % sodium glutamate
feeding rate; 12 ml/hour
addition of caffeine; after 9 hour culture, caffeine was added in order to maintain the caffeine concentration in the range of 0.5 mg/ml to 2.5 mg/ml with monitoring the caffeine concentration in the culture broth by HPLC.

As a result of the cultivation as described above, 165 g of caffeine was introduced and 133 g of theobromine was produced. The concentrations of theobromine and caffeine at the termination of the culture was 39.8 g/l and 0.3 g/l, respectively. Ninety-nine percents or more of the caffeine introduced were consumed and the conversion rate of caffeine into theobromine was 88 %.

The present invention provides a mutant bacterial strain capable of constitutively converting caffeine into theobromine and genetically deficient in the system for the demethylation of theobromine, obtained by inducing mutations in a microorganism having a potency of converting caffeine into theobromine. The present invention also provides a method to efficiently produce theobromine using the above microorganism.

TABLE 1

| Exp. No. | Nickel ion | Magnesium sulfate | Amount of remaining caffeine | Yield of theobromine |
|---|---|---|---|---|
| 1 | added | added | 8.24 g/l | 17.7 g/l |
| 2 | not added | added | 0.52 | 8.26 |
| 3 | added | not added | 20.2 | 8.63 |
| 4 | not added | not added | 13.6 | 5.67 |

## Claims

1. A mutant strain from the genus <u>Pseudomonas</u>, said mutant strain being capable of converting caffeine into theobromine and substantially lacking an ability to metabolize theobromine.

2. A mutant strain from the genus <u>Pseudomonas</u>, said mutant strain being capable of converting caffeine into theobromine, wherein theobromine conversion is not under the influence of the nickel ion.

3. A method for converting caffeine into theobromine, using a mutant strain from the genus <u>Pseudomonas</u>, wherein said mutant strain is capable of converting caffeine into theobromine and substantially lacking an ability to metabolize theobromine.

4. A method for converting caffeine into theobromine using a mutant strain from the genus <u>Pseudomonas</u>, wherein theobromine conversion of the mutant is not under the influence of the nickel ion.